# FASCICULE DE BREVET EUROPEEN

(11) **EP 1 950 210 B1**
(45) Date de publication et mention de la délivrance du brevet: **16.01.2013**
(21) Numéro de dépôt: 07001384.2
(22) Date de dépôt: 23.01.2007
(51) Int. Cl.: C07D 311/62, A23D 9/06, A23L 3/3544, C09K 15/08, C12P 17/06

(54) **Utilisation de dérivés polyphénoliques liposolubles comme antioxydants**
Verwendung von fettlöslichen Polyphenolderivaten als Antioxidanten
Use of fat-soluble polyphenolic derivatives as antioxidants

(43) Date de publication de la demande: 30.07.2008
(73) Titulaire: Polaris SAS, 29170 Pleuven (FR)
(72) Inventeur: Breton, Gildas, 29561 Quimper Cedex (FR)
(74) Mandataire: Célanie, Christian

(56) Documents cités:
- EP-A- 0 618 203
- WO-A-01/79245
- WO-A-99/22728
- FR-A- 2 893 026
- KUMAGAI A ET AL: "Tumor chemopreventive activity of 3-O-acylated (-)-epigallocatechins" BIOORGANIC & MEDICINAL CHEMISTRY, vol. 11, no. 23, 2003, pages 5143-5148, XP002390170
- HIIPAKKA R A ET AL: "Structure-activity relationships for inhibition of human 5-alpha-reductases by polyphenols" BIOCHEMICAL PHARMACOLOGY, vol. 63, no. 6, 2002, pages 1165-1176, XP002242332
- UESATO S ET AL: "Antitumor promoting activities of 3-O-acyl-(-)-epigallocatechins" BIOORGANIC & MEDICINAL CHEMISTRY LETTERS, vol. 10, no. 15, 7 août 2000 (2000-08-07), pages 1673-1675, XP004213221
- ARMESTO N ET AL: "Regioselective enzymatic acylation of methyl shikimate. Influence of acyl chain length and solvent polarity on enzyme specificity" JOURNAL OF ORGANIC CHEMISTRY, vol. 67, no. 14, 12 juin 2002 (2002-06-12), pages 4978-4981, XP002974845

## Description

La présente invention se rapporte aux nécessités de la vie, plus particulièrement à l'alimentation, à la nutrition et aux compléments alimentaires.

La description décrit un procédé de préparation de dérivés polyphénoliqucs liposolubles, qui consiste à estérifier ou transestérifier des dérivés catéchiques par un dérivé fonctionnel d'acide gras en présence d'un catalyseur.

L'invention a spécifiquement pour objet l'utilisation de ces dérivés polyphénoliques comme antioxydants, notamment pour stabiliser des acides gras insaturés ou polyinsaturés, en particulier dans les huiles contenant ce type d'acides gras, comme les huiles animales ou végétales (huiles de poisson, de bourrache, de lin, de pépin de kiwi).

Le problème s'est posé en effet de trouver un agent de stabilisation protégeant les acides gras insaturés, notamment ceux des séries n-3 et n-6, contre les peroxydations et les oxydations liées à la présence d'oxygène. Ces réactions conduisent en effet à des composés inutilisables, notamment à des résines polymères.

Un procédé existant permet la protection des huiles ou des corps gras contenant des acides gras insaturés, par incorporation dans un agent d'enrobage en formant un clathrate. Un tel procédé est peu économique car la quantité adsorbée est faible (moins de 5%) et ne peut être augmentée (EP0796238).

Un autre procédé consiste à ajouter aux huiles ou corps gras, des polyphénols ou des caroténoïdes susceptibles de réduire les phénomènes d'oxydation. Ces antioxydants ont le défaut d'être peu lipophiles, donc peu miscibles aux huiles.

Les extraits de thé vert, riches en polyphénols, ont de nombreuses propriétés bénéfiques, notamment antioxydantes. Leur utilisation est très répandue dans des préparations pharmaceutiques, cosmétiques ou nutritionnelles. Ils ont par exemple été employés à la lutte contre l'hypertension (EP1214085) et contre l'amyloidose dans la maladie d'Alzheimer (EP1246632). Ils ont également été utilisés dans le traitement des peaux sèches (EP1082100) et dans des produits bronzants (EP1089707). Enfin, ils ont été introduits dans de nombreuses préparations alimentaires, notamment des boissons (EP0416667).

Les catéchines sont des polyphénols abondamment présents dans les extraits de thé vert. Elles sont très solubles dans l'eau mais insolubles dans les graisses. Cette propriété a longtemps freiné l'utilisation comme antioxydants de préparations alimentaires lipidiques, comme par exemple les huiles animales ou végétales, notamment les huiles riches en acides gras polyinsaturés de type Oméga-3 ou Oméga-6.

Le brevet WO94/22321 (Kalamazoo Holdings) décrit la préparation de solutions liposolubles à base d'extrait de thé vert. Cette invention consiste à solubiliser les catéchines dans un solvant adapté (lécithine, alcool à longue chaîne, agent de surface...), le mélange obtenu étant liposoluble. Toutefois, aucune transformation chimique des catéchines n'intervient dans cette opération. L'inconvénient de cette invention est qu'elle nécessite l'introduction d'importantes quantités d'un solvant liposoluble en même temps que l'antioxydant.

Le brevet WO94/22322 (Kalamazoo Holdings) décrit, en outre, la fabrication d'antioxydants à base de microparticules de catéchines extraites du thé vert. Celles-ci sont incorporées dans les lipides sous forme de suspension ou de dispersion. L'activité antioxydante des catéchines microcristallisées est toutefois moins importante que celle des catéchines solubilisées.

Le brevet EP703954 (Berkem) décrit l'estérification de composés polyphénoliques extraits de diverses sources végétales, notamment du thé vert. Toutefois, le procédé utilisé conduit à l'estérification d'une grande partie des -OH phénoliques. Or, ce sont ces groupements qui interviennent dans la capture de l'oxygène et/ou des radicaux libres, conférant à la molécule ses propriétés antioxydantes. Une catéchine dont les groupements phénoliques sont protégés par un ester perd la majeure partie de son activité antioxydante.

Afin de rendre liposolubles des dérivés polyphénoliques, sans nuire à leurs propriétés antioxydantes, il est possible de réaliser des dérivés monoestérifiés. Le brevet EP618203 (Mitsui Norin Co.) référence la plus proche décrit la formation de catéchines 3-O-acylées par l'intermédiaire d'une carboxylestérase. Toutefois, cette méthode ne permet d'introduire que des chaînes carbonées relativement courtes, ce qui limite la liposolubilité des esters catéchiques obtenus. Ces derniers ne pourront être introduits dans les huiles qu'en faible quantité et l'effet antioxydant atteint ne sera pas toujours suffisant.

Il était donc souhaitable d'utiliser des dérivés de catéchines associant une excellente liposolubilité à une activité antioxydante non amoindrie.

La présente description décrit un procédé de production d'une forme monoestérifiée de catéchines, notamment extraites du thé vert, par un groupe fonctionnel à longue chaîne carbonée. Les composés obtenus sont très liposolubles tout en conservant leur activité antioxydante ; ils peuvent être incorporés aux huiles de façon stable et efficace et empêcher ainsi les phénomènes d'oxydation ou de polymérisation.

La description décrit en particulier un procédé de production d'esters de polyphénols répondant à la formule (I) : 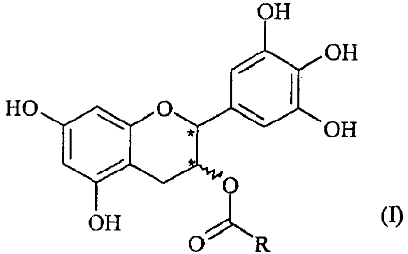 dans laquelle R est une chaîne alkyle, linéaire ou ramifiée, pouvant comporter une ou des insaturations et/ou un ou des groupements -OH et comptant de 10 à 25 atomes de carbone. La ligne ondulée indique une configuration *cis* ou *trans* des deux carbones asymétriques, signalés par un astérisque.

Les fonctions phénol peuvent en outre être engagées dans une liaison osidique.

Selon une forme préférentielle, R est une chaîne alkyle saturée, linéaire ou ramifiée, comportant de 10 à 22 atomes de carbone.

Selon une autre forme préférentielle, R est une chaîne alkényle, linéaire ou ramifiée, comportant de 10 à 25 atomes de carbone et de 1 à 5 doubles liaisons.

Selon une autre forme préférentielle, R est une chaîne alkyle, linéaire ou ramifiée, comportant de 10 à 25 atomes de carbone et de 1 à 3 groupements -OH.

Ces nouveaux composés sont obtenus par une réaction d'estérification, entre des dérivés d'acides gras et des polyphénols de type catéchines, plus particulièrement la gallocatéchine ou l'épigallocatéchine, sous forme libre ou estérifiée par l'acide gallique.

Les catéchines sont préférentiellement extraites du thé vert. En effet, les catéchines extraites des pommes, du raisin, du cacao ou de différentes écorces contiennent des mélanges complexes de catéchines polymérisées. L'intérêt des extraits de thé est de contenir essentiellement des catéchines monomères.

L'extrait de thé vert utilisé peut être obtenu selon un procédé d'extraction constitué des étapes suivantes : des feuilles de thé vert subissent une extraction à l'eau déionisée, à une température d'environ 50 °C. Le mélange est centrifugé, la phase liquide supérieure est récupérée puis cette phase est extraite par un solvant organique à environ 40 °C. Le solvant est ensuite évaporé sous pression réduite pour obtenir un résidu solide concentré en polyphénols. Une deuxième phase d'extraction à l'eau déionisée / extraction par solvant organique permet une meilleure purification. A l'issue de l'évaporation des solvants, le résidu solide est réduit en poudre.

Selon une forme préférentielle de l'invention, l'extrait de thé vert utilisé possède le profil en catéchines suivant :

| | |
|---|---|
| Epigallocatéchine (EGC) : | 9-17 % |
| Epigallocatéchine-gallate (EGC-g) : | 52-59 % |
| Gallocatéchine-gallate (GC-g) : | 2-6 % |
| Epicatéchine (EC) : | 5-6 % |
| Epicatéchine-gallate (EC-g) : | 10-25 % |
| DL-Catéchine (DL-C) : | 2-6 % |

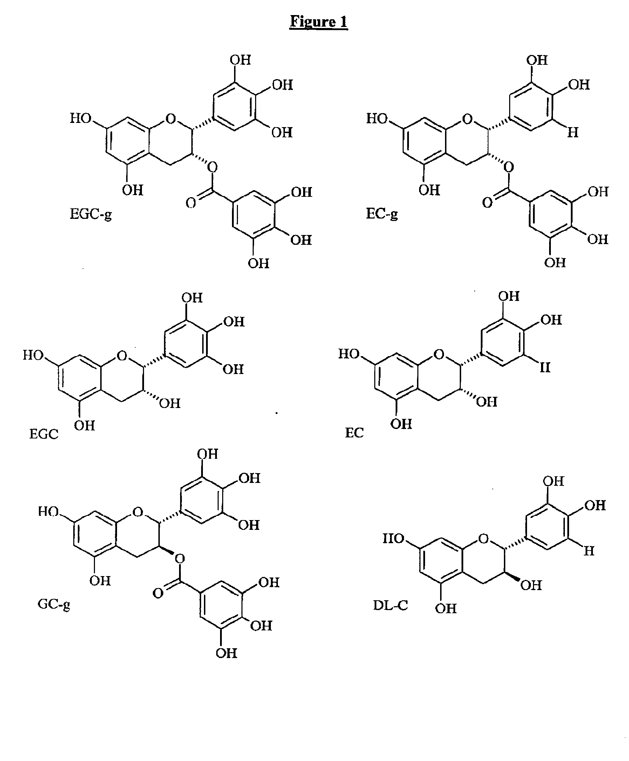

Les formules de ces différents composés sont représentées à la figure 1.

Selon une forme préférentielle, la teneur en polyphénols de cet extrait est de 90% minimum.

En outre, les catéchines sont estérifiées par un acide gras ou un dérivé fonctionnel d'acide gras, comme par exemple un chlorure d'acyle, un anhydride ou un ester.

Des exemples d'acide gras sont donnés ci-après, la liste étant non limitative. Selon la nomenclature classique, le nombre d'atomes de carbone est indiqué, suivi du nombre de doubles liaisons et de l'emplacement de ces doubles liaisons sur la chaîne carbonée. Les noms des acides gras sont précisés pour les plus classiques.

Il s'agit des acides undécylénique C11:1 ; C11:2 ; laurique C12:0 ; C13:0 ; C13:2 ; myristique C14:0 ; C15:0 ; C15:2 ; palmitique C16:0 ; C16:1(n-7) ; C16:2(n-4) ; C16:2(n-7) ; C16:3(n-4) ; C16:4 ; C17:0 ; stéarique C18:0 ; oléïque C18:1(n-9) ; C18:1(n-7) ; linoléique C18:2(n-6) ; γ-linolénique C18:3(n-6) ; α-linolénique C18:3(n-3) ; stéaridonique C18 :4(n-3) ; C20:0 ; C20:1(n-9) ; C20:2(n-6); dihomo-y-linolénique C20:3(n-6); C20:4(n-6) ; arachidonique C20:4(n-3) ; eicosapentaénoïque C20:5(n-3) ; béhénique C22:0; C22:1 ; C22:1(n-5) ; C22:3(n-3) ; C22:4(n-6) ; C22:4(n-3) ; C22:5(n-3) ; C22:5(n-6) ; docosahexaénoïque C22:6(n-3); C24:1(n-9) ; brassylique (HO₂C(CH₂)₁₁CO₂H) ; ricinoléique (CH₃(CH₂)₅CH-(OH)CH₂-CH=CH-(CH₂)₇CO₂H) ; hydroxystéarique (CH₃(CH₂)₅CH(OH)(CH₂)₁₀CO₂H) ; hydroxymyristique.

Selon une forme préférée, les acides gras ou les dérivés d'acides gras utilisés sont les acides ou les dérivés d'acides stéarique, palmitique et/ou myristique.

Les composés de formule (1) sont synthétisés par le couplage d'un polyphénol de type catéchine ou d'un mélange de polyphénols de type catéchine, avec un acide gras ou un dérivé d'acide gras, en présence d'un catalyseur enzymatique de type lipase.
L'enzyme peut être libre ou immobilisé sur un support inerte.

Selon une forme préférentielle, le procédé de couplage consiste en une transestérification d'un ester d'acide gras par un polyphénol ou un mélange de polyphénols de type catéchine, en présence d'un catalyseur enzymatique de type lipase.

Selon une forme préférentielle, l'enzyme utilisé est une lipase de *Candida antartica,* notamment celle commercialisée sous la marque Novozym® 435. Cette lipase est issue de la fermentation d'un *Aspergillus* génétiquement modifié.

Les groupements phénoliques ayant peu d'affinité pour les lipases, l'estérification s'effectue sélectivement sur l'alcool secondaire des catéchines. Dans le cas de EGC-g, EC-g et GC-g, la fonction alcool est estérifiée par un groupement gallate. Une transestérification directe s'effectue alors avec l'acide gras ou le dérivé d'acide gras. A la fin de la réaction, il ne reste quasiment plus de catéchine liée à l'acide gallique.

Un excès d'ester d'acide gras joue le rôle de solvant. Un co-solvant peut être apporté pour améliorer la dissolution ou la dispersion des polyphénols. Ce co-solvant doit être non réactif vis-à-vis de l'estérification et suffisamment polaire pour solubiliser les polyphénols. Par exemple, un alcool tertiaire, un hydrocarbure ou une cétone aliphatiques, ou encore un éther dialiphatique, peuvent être utilisés.

Une agitation vigoureuse est nécessaire afin d'homogénéiser les deux phases et d'apporter un maximum de substrat disponible à l'enzyme.

La réaction s'effectue à chaud, préférentiellement entre 50 et 85 °C.

La réaction s'effectue avantageusement sous pression réduite, afin d'éliminer l'alcool formé et de déplacer l'équilibre dans le sens de la synthèse. Une gamme de pression préférentielle est 200-250 mbars.

Selon une forme préférentielle de la synthèse, l'ester d'acide gras utilisé est un ester alkylique, par exemple méthylique, éthylique, propylique, isopropylique ou butylique. L'alcool résiduel est éliminé au fur et à mesure de la réaction, entre 50 et 85°C, sous une pression choisie en fonction de son point d'ébullition. Cette élimination permet de déplacer l'équilibre dans le sens de la synthèse et d'obtenir des rendements élevés.

Selon une forme préférentielle, le mélange réactionnel est ensuite soumis au traitement constitué des étapes suivantes : après une filtration visant à éliminer l'enzyme, le co-solvant éventuellement présent est distillé. Le résidu est ensuite repris dans un solvant apolaire, comme par exemple l'hexane, dans lequel les esters catéchiques précipitent. Après filtration, le précipité est lavé par un solvant alcoolique qui est également filtré. Les traces de solvant sont éliminées par évaporation afin d'obtenir une poudre d'esters de catéchines.

Il est possible d'éliminer l'acide gallique résiduel par lavages successifs à l'aide d'une solution aqueuse alcaline. Toutefois, dans une forme préférentielle de l'invention, ces lavages ne se sont pas avérés nécessaires.

D'autres méthodes physiques de séparation des esters peuvent être utilisées, comme par exemple la distillation moléculaire en couche mince.

D'une manière préférée, le produit final sous forme de poudre contient au minimum 90% de polyphénols et au moins 85% de catéchines. Plus de 80% de ces catéchines sont des (esters d'épi et/ou)gallocatéchines et d'acide gras, de formule générale (I) : 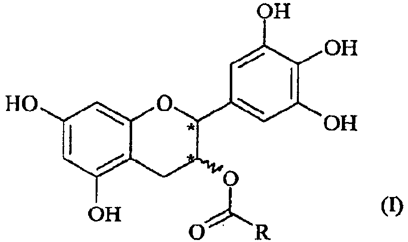 dans laquelle R est défini comme précédemment.

Les propriétés antioxydantes des composés préparés sont avantageusement mises à profit pour la stabilisation de préparations alimentaires, nutritionnelles ou diététiques.

L'invention consiste en conséquence à utiliser les esters de polycatéchines définis comme ci-dessus comme agents antioxydant et stabilisant des huiles, selon la revendication 1.

Elles sont notamment incorporées à des huiles animales ou végétales, riches en acides gras polyinsaturés, notamment de type Oméga-3 ou Oméga-6.

Une utilisation essentielle réside dans la stabilisation des huiles de poisson (thon, sardine...).

Pour une utilisation en tant qu'antioxydant dans une huile, il est préférable de dissoudre la poudre, à hauteur de 20 à 40%, dans une huile neutre saturée, comme par exemple une huile triglycéride en C8-C10. La dissolution s'effectue préférentiellement à chaud (entre 70 °C et 90 °C). Plus le groupement R contient un nombre élevé de carbones, plus une température élevée est nécessaire. Une fois l'opération de solubilisation effectuée, le mélange est stable, même à basse température.

Ce mélange peut alors être incorporé à l'huile à stabiliser. Une quantité préférentielle se situe entre 3.000 et 7.000 ppm, soit entre 1.000 et 2.000 ppm environ de principe actif. Toutefois, en cas de nécessité, l'excellente solubilité du mélange dans les huiles permet une incorporation en quantité bien supérieure (comme par exemple 10.000 ppm de principe actif) afin d'obtenir une meilleure protection contre les phénomènes d'oxydation.

L'addition de la solution de polyphénols à l'huile polyinsaturée s'effectue de préférence entre 25 et 30 °C, sous atmosphère inerte (azote ou argon) afin d'éviter toute incorporation d'oxygène dans l'huile.

Dans ces conditions, la solubilité des catéchines estérifiées est excellente. Après un stockage de plusieurs jours à une température inférieure à 0 °C, aucun précipité n'est apparu dans les huiles testées.

Les produits ainsi obtenus se conservent dans le temps d'une manière très satisfaisante. Ils présentent une stabilité élevée aux phénomènes de rancissement et de polymérisation.

Les exemples suivants illustrent l'invention sans toutefois la limiter :

### Exemple 1 : Formation de stéarate d'(épi et/ou)gallocatéchine

- Enzyme utilisé : Lipase de *Candida antartica* immobilisée sur une résine acrylique macroporeuse / Novozym® 435 Type B (Novo Nordisk).
- Extrait de thé vert : Tecalon - 90S (Quimdis France) :

| | |
|---|---|
| Total polyphénols | 96 % |
| Total catéchines | 79,2 % |
| Epigallocatéchine (EGC) | 11,1 % |
| Epigallocatéchine-gallate (EGC-g) | 42,5 % |
| Epicatéchine (EC) | 4,9 % |
| Epicatéchine-gallate (EC-g) | 13,8 % |
| Gallocatéchine-gallate (GC-g) | 3,8 % |
| DL-Catecltine (DL-C) | 2,5 % |

La réaction catalytique est effectuée dans un réacteur double enveloppe de 1,5 L, sous une pression de 250 mbars et sous agitation vigoureuse. L'équivalent de 3 g de catéchines est mélangé à 700 g de stéarate d'éthyle à une température de 70 °C. L'enzyme sur résine est introduit à hauteur de 25 g/L.

Après 12 heures de réaction, une chromatographie sur couche mince indique que plus de 90% des catéchines sont estérifiées. Le mélange réactionnel est alors filtré, puis les esters de catéchines sont précipités par ajout de quatre volumes d'hexane. Après filtration, le précipité est lavé par un solvant alcoolique qui est lui aussi filtré. Les traces de solvant sont éliminées par évaporation sous vide.
Rendement : 90% environ

### Exemple 2 : Formation de palmitate d'(épi et/ou)gallocatéchine

La réaction s'effectue dans les mêmes conditions qu'à l'exemple 1, en utilisant du palmitate d'éthyle au lieu de stéarate d'éthyle.
Rendement : 90% environ

### Exemple 3 : Formation de myristate d'(épi et/ou)gallocatéchine

La réaction s'effectue dans les mêmes conditions qu'à l'exemple 1, en utilisant du myristate d'éthyle au lieu de stéarate d'éthyle.
Rendement : 90% environ

### Exemple 4 : Evaluation des propriétés antioxydantes des esters de catéchine

Les propriétés antioxydantes des esters de catéchines selon l'invention ont été évaluées à partir du test Rancimat™. La méthode consiste à mesurer les variations de conductivité d'une eau distillée dans laquelle sont recueillis les acides de faible masse, comme l'acide formique, produits lorsque l'huile à analyser est soumise à des températures élevées sous balayage d'air.

L'appareil utilisé est un Rancimat 679 (Methrom). La température de mesure est fixée selon le cas à 80 °C, 98 °C ou 110 °C, le débit d'air est réglé à 20 litres/h et la prise d'essai est fixée à 3 grammes afin de permettre aux tubulures d'arrivée d'air de plonger dans l'échantillon pendant toute la durée de l'expérimentation.

Le tracé de la conductivité (κ) en fonction du temps (t) est obtenu sur enregistreur et la mesure du temps de résistance ou temps d'induction (point d'inflexion) est directe. Ce temps d'induction correspond au temps à partir duquel les réactions d'oxydation commencent à se propager dans l'huile ; il est la caractéristique de la stabilité à l'oxydation de l'échantillon analysé. Il permet de déterminer le facteur de protection (temps d'induction de l'antioxydant / temps d'induction témoin) lié à chaque antioxydant.

### - Analyse de la stabilité d'une huile de bourrache :

La stabilité à l'oxydation de deux échantillons d'huile de bourrache a été comparée. L'huile contient 20% d'acide γ-linolénique (GLA), appartenant à la famille des Oméga-6. Le premier échantillon ne contient pas d'antioxydant, le second (huile de bourrache QS) contient une solution de stéarate d'(épi et/ou)gallocatéchine selon l'invention, à hauteur de 1.500 ppm de principe actif.

**Tableau 1 : Temps d'induction sur une huile de bourrache - Test Rancimat (80 °C, 20 L/h air)**

| Echantillons | Temps d'induction (heures) |
|---|---|
| Huile de bourrache 20% GLA | 24,4 |
| Huile de bourrache QS | 186,5 |

La présence d'antioxydant conduit à un allongement du temps d'induction d'un facteur 7,6, soit à une très bonne amélioration de la résistance aux phénomènes d'oxydation et de peroxydation.

### - Analyse de la stabilité d'une huile de poisson :

La stabilité à l'oxydation de plusieurs échantillons d'huile de poisson a été comparée. L'huile contient 18% d'EPA (acide eicosapentaénoïque C20:5(n-3)) et 12% de DHA (acide docosahexaénoïque C22:6(n-3)). Ces deux acides gras appartiennent à la série des Oméga-3. Chaque échantillon d'huile contient un antioxydant différent.

**Tableau 2 : Facteur de protection (FP) à 1.500 ppm d'antioxydant, sur une huile de poisson contenant 18% EPA / 12% DHA - Test Rancimat (80 °C, 20 L/h air)**

| Antioxydant | FP |
|---|---|
| Concentrât naturel de tocophérols (Jan Dekker) | 2,45 |
| Extrait végétal riche en d-alpha tocophérols (Cognis) | 2,29 |
| Extrait de romarin (Naturex) | 1,65 |
| Extrait de romarin (Sensient) | 1,74 |
| Solution riche en stéarate d'(épi et/ou)gallocatéchine selon l'invention | 5,65 |

Le facteur de protection de la solution selon l'invention est significativement supérieur à celui d'autres antioxydants classiquement utilisés. On peut notamment remarquer qu'il est 2,3 fois supérieur à celui du concentrât de tocophérols,

## Revendications

1. Utilisation d'esters de polyphénols répondant à la formule générale 1 : 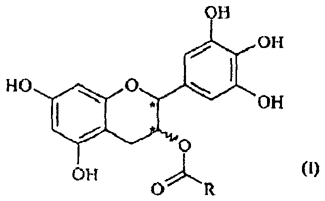 dans laquelle R est une chaine alkyle, linéaire ou ramifiée, pouvant comporter une ou des insaturations et/ou un ou des groupements -OH, et comportant entre 10 et 25 atomes de carbone, la ligne ondulée indiquant une configuration cis ou trans de deux carbones asymétriques signalés par un astérisque comme agents antioxydants pour la stabilisation d'huiles animales ou végétales riches en acides gras polyinsaturés.

2. Utilisation des esters de polyphénols selon la revendication 1, pour la stabilisation des huiles de poisson comme par exemple l'huile de thon ou l'huile de sardine.

3. Utilisation des esters de polyphénols selon la revendication 1 ou 2, dans laquelle on dissout la poudre d'esters de polyphénols dans une huile neutre saturée à hauteur de 20 à 40% pour obtenir une solution d'esters de polyphénols, de préférence à chaud, puis on incorpore cette solution dans l'huile à stabiliser.

4. Utilisation des esters de polyphénols selon la revendication 3, dans laquelle on incorpore la solution d'esters de polyphénol dans l'huile à stabiliser à une concentration variant de 1000 à 2000 ppm environ de principe actif.

5. Utilisation des esters de polyphénols selon la revendication 3 ou 4, comme agent antioxydant, consistant à additionner la solution d'esters de polyphénols à une huile polyinsaturée entre 25 et 30°C sous atmosphère inerte.

6. Utilisation des esters de polyphénols selon l'une des revendications précédentes, comme agents antioxydants dans lesquels ledit ester de polyphénol est un stéarate d'(épi et/ou) gallocatéchine.

7. Utilisation des esters de polyphénols selon l'une des revendications précédentes, comme agents antioxydants dans lesquels ledit ester de polyphénol est un palmitate d'(épi et/ou) gallocatéchine.

8. Utilisation des esters de polyphénols selon l'une des revendications précédentes, comme agents antioxydants dans lesquels ledit ester de polyphénol est un myristate d'(épi et/ou) gallocatéchine.

## Claims

1. Use of polyphenol esters according to the general formula 1: 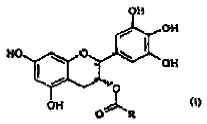 wherein R is an alkyl chain, straight or branched, that may incorporate one or more unsaturations and/or -OH groups, and incorporating between 10 and 25 carbon atoms, the wavy line indicating a cis or trans configuration of two asymmetrical carbons signalled by an asterisk as anti-oxidants for the stabilization of animal or vegetable oils rich in polyunsaturated fatty acids.

2. Use of polyphenol esters according to Claim 1, for the stabilization of fish oils, for example tuna oil or sardine oil.

3. Use of polyphenol esters according to Claims 1 or 2, in which the polyphenol ester powder is dissolved, preferably heated, in a neutral oil saturated at 20 to 40 percent to obtain a polyphenol ester solution, this solution then being incorporated into the oil to be stabilized.

4. Use of polyphenol esters according to Claim 3, in which the polyphenol ester solution is incorporated into the oil to be stabilized at a concentration that varies between 1,000 to 2,000 ppm approximately of active substance.

5. Use of polyphenol esters according to Claims 3 or 4, as an anti-oxidant, consisting in adding the polyphenol ester solution to a polyunsaturated oil at between 25 and 30°C in an inert atmosphere.

6. Use of polyphenol esters according to one of the above Claims, as anti-oxidants in which said polyphenol ester is an epi- and/or gallocatechine stearate.

7. Use of polyphenol esters according to one of the above Claims, as anti-oxidants in which said polyphenol ester is an epi- and/or gallocatechine palmitate.

8. Use of polyphenol esters according to one of the above Claims, as anti-oxidants in which said polyphenol ester is an epi- and/or gallocatechine myristate.

## Patentansprüche

1. Verwendung von Polyphenolestern, die der allgemeinen Formel 1 entsprechen,: 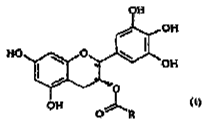 in welcher R eine lineare oder verzweigte Alkylkette ist, welche eine oder mehrere Ungesättigtheiten und / oder eine oder mehrere -OH Gruppen umfassen kann und zwischen 10 und 25 Kohlenstoffatomen umfasst, wobei die gewellte Linie eine cis- oder trans-Konfiguration von zwei asymmetrischen Kohlenstoffen angibt, die durch ein Sternchen gekennzeichnet sind, als Antioxidansmittel zur Stabilisierung von tierischen oder pflanzlichen Ölen, die reich an mehrfach ungesättigten Fettsäuren sind.

2. Verwendung von Polyphenolestern nach Anspruch 1 zur Stabilisierung von Fischölen, wie zum Beispiel Thunfischöl oder Sardinenöl.

3. Verwendung von Polyphenolestern nach Anspruch 1 oder 2, bei welcher das Pulver von Polyphenolester in einem neutralen, im Bereich von 20 bis 40 % gesättigten Öl aufgelöst wird, um eine Polyphenolester-Lösung, vorzugsweise unter Hitze, zu erhalten, und dann diese Lösung in das zu stabilisierende öl beigemengt wird.

4. Verwendung von Polyphenolestern nach Anspruch 3, bei welcher die Polyphenolester-Lösung dem zu stabilisierenden öl mit einer Konzentration beigemengt wird, welche zwischen ungefähr 1000 und 2000 ppm des Wirkstoffs variiert.

5. Verwendung von Polyphenolestern als Antioxidansmittel nach Anspruch 3 oder 4, welche darin besteht, dass die Polyphenolester-Lösung einem mehrfach ungesättigten Öl zwischen 25 und 30°C unter Inertatmosphäre zugefügt wird.

6. Verwendung von Polyphenolestern als Antioxidansmittel nach einem der vorhergehenden Ansprüche, bei welchen der genannte Polyphenolester ein Stearat von (Epi- und / oder) Gallocatechin ist.

7. Verwendung von Polyphenolestern als Antioxidansmittel nach einem der vorhergehenden Ansprüche, bei welchen der genannte Polyphenolester ein Palmitat von (Epi- und / oder) Gallocatechin ist.

8. Verwendung von Polyphenolestern als Antioxidansmittel nach einem der vorhergehenden Ansprüche, bei welchen der genannte Polyphenolester ein Myristat von (Epi- und / oder) Gallocatechin ist.
